# EUROPEAN PATENT APPLICATION

(11) **EP 4 644 563 A1**
(43) Date of publication of application: **05.11.2025**
(21) Application number: 22969504.4
(22) Date of filing: 26.12.2022
(51) Int. Cl.: C12Q 1/68, C12Q 1/6806, C12Q 1/682, C12Q 1/6841, C12Q 1/6869, C12Q 1/6874

(54) **IN SITU SEQUENCING LIBRARY CONSTRUCTION METHOD, IN SITU SEQUENCING METHOD, AND USE**

(71) Applicant: BGI Shenzhen, Shenzhen, Guangdong 518083 (CN); BGI Shenzhen Co., Ltd, Shenzhen, Guangdong 518083 (CN)
(72) Inventor: HE, Ying, Shenzhen, Guangdong 518083 (CN); CHEN, Ao, Shenzhen, Guangdong 518083 (CN); LIAO, Sha, Shenzhen, Guangdong 518083 (CN); ZHANG, Wenwei, Shenzhen, Guangdong 518083 (CN)
(74) Representative: Ter Meer Steinmeister & Partner
(86) International application number: PCT/CN2022/142127
(87) International publication number: WO 2024/138347

(57) **Abstract**

Provided are a method for constructing an *in situ* sequencing library, and an *in situ* sequencing method and an application. The construction method includes: placing a sample on a solid support having an RNA capture function, and then performing non-crosslinking fixation and capturing on mRNA in the tissue sample or cell sample; reverse transcribing the captured mRNA into cDNA, and removing residual tissue in the reverse-transcribed tissue sample or residual cells in the reverse-transcribed cell sample, thereby obtaining a solid support with impurities removed, wherein the cDNA is immobilized on the solid support with impurities removed; and performing target capture and amplification on the cDNA on the solid support with impurities removed, thereby obtaining an amplification product serving as an *in situ* sequencing library. By designing a capture probe on the solid support to bind to the mRNA in the sample, the mRNA is immobilized on the solid support, which does not require a chemical cross-linking reagent to fix the mRNA in the sample, thereby avoiding the problem of low reverse transcription efficiency caused by a cross-linking fixation operation.

## Description

### Technical Field

The present invention relates to the technical field of sequencing, and specifically, to a method for constructing an *in situ* sequencing library, and an *in situ* sequencing method and an application.

### Background

An *in situ* sequencing technology originated in 2013 is a targeted transcriptome sequencing technology. The key to this technology lies in discovering a correlation between sequencing information and positions within a tissue or cell sample(e.g., subcellular localization). The *in situ* sequencing method relies on a padlock probe that hybridizes to either end of a target sequence, thereby forming a circular template for replication. Since a product is tied to the template, which provides reliable localization, and then *in situ* sequencing is realized by continuous introduction of oligonucleotide probes. Through the method, sequencing is directly performed on mRNA in a section of a fixed tissue or cell sample.

After nearly a decade of development, the *in situ* sequencing technology has been widely used in research such as developmental biology, neurobiology, oncology, etc. A more mature *in situ* sequencing technology currently on the market is hybridization sequencing-based *in situ* sequencing technology (HyblSS) of 10X genomics. In addition to the HybISS, there are also ISS, BaristaSeq, STARmap, and FISSQ. In these technologies, instead of using probes for targeted detection, the FISSQ uses a method similar to a next-generation sequencing technology to directly reverse transcribe RNA into cDNA *in situ* followed by circularization and amplification, and then directly perform *in situ* sequencing of longer fragments in the cell or tissue. Other *in situ* sequencing technologies realize single-molecule amplification of a targeting sequence based on a Rolling Circle Amplification (RCA) reaction of a targeting probe, thereby using the principle of sequencing to obtain genetic information by decoding different color signals.

However, at present, the prior art has the following disadvantages: first, all the above technologies are required to fix the RNA of the cell/tissue sample in the cell by cross-linking, that is to say, the cell/tissue sample is treated by formaldehyde or PFA, and a covalent bond is formed between the protein and nucleic acid in the sample, thereby "freezing" the interacting state of the protein and the nucleic acid. However, cross-linking fixation will damage the sample and reduce the efficiency of reverse transcription, thereby affecting the sensitivity and accuracy of detection. Second, during steps of reagent application, washing, etc., the persistence of tissue sample may affect the efficiency of the reaction and increase the interference of background fluorescence. Furthermore, sequencing-related dyes also tend to linger in the tissue to produce adverse effect on final results. Third, since the tissue or cell is fixed on a slide, a highly technical microfluidic operation is required during a plurality of rounds of sequencing reactions as well as imaging, leading to great inconvenience to the operation.

### Summary

The present invention is mainly intended to provide a method for constructing an *in situ* sequencing library, and an *in situ* sequencing method and an application, thereby solving the problem in the prior art of low efficiency of reverse transcription reactions when an *in situ* sequencing library is constructed.

In order to implement the above objective, a first aspect of the present invention provides a method for constructing an *in situ* sequencing library. The method comprises: S1, placing a sample on a solid support having an mRNA capture function, and then performing non-crosslinking fixation and capture on mRNA in the sample, wherein the sample is a tissue sample or a cell sample; S2, reverse transcribing the captured mRNA into cDNA, and removing residual tissue in the reverse-transcribed tissue sample or residual cells in the reverse-transcribed cell sample, thereby obtaining a solid support with impurities removed, wherein the cDNA is immobilized on the solid support with impurities removed; and S3, performing target capture and amplification on the cDNA on the solid support with impurities removed, thereby obtaining an amplification product, wherein the amplification product is an *in situ* sequencing library.

Further, the sample is a section sample.

Further, the section sample is a tissue section or a cell section.

Further, in S1, the solid support having the mRNA capture function is a solid support with surface-modified Poly T clusters.

Further, in S1, the non-crosslinking fixation means that the sample is fixed on the solid support without using a cross-linking agent.

Further, the cross-linking agent is a carbonyl compound, a N-hydroxysuccinimide ester compound, an imidoester compound, a maleimide compound, a halogenated acetyl compound, a dithiodipyridine compound, and/or a carbodiimide compound.

Further, in S2, the residual tissue in the reverse-transcribed tissue sample or the residual cells in the reverse-transcribed cell sample are removed with Proteinase K.

Further, the solid support is a silicon-based planar support.

Further, S1 includes: S11, attaching the sample to a solid support with surface-modified Poly T clusters, thereby obtaining a mounted sample; and S12, performing permeabilization treatment on the mounted sample to release the mRNA in the sample, and capturing the mRNA in the sample on the solid support through the Poly T cluster.

Further, S2 includes: S21, reverse transcribing the mRNA into the cDNA using the Poly T cluster as a primer; and S22, incubating the sample on the solid support with a tissue removal solution, to remove the residual tissue in the reverse-transcribed tissue sample or the residual cells in the reverse-transcribed cell sample, thereby obtaining the solid support with impurities removed, wherein the cDNA is immobilized on the solid support with impurities removed.

Further, between S21 and S22, the method further includes S21' of washing residual reverse transcription-related reagents from the reverse-transcribed tissue sample or reverse-transcribed cell sample.

Further, the tissue removal solution with a pH of 8.0 includes 200-500 mM Tris, 10-20 mM EDTA, 5%-10% Triton X-100, 200-500 mM NaCl, and 5-20 U/ml Proteinase K; alternatively, the tissue removal solution with a pH of 8.0 includes 200-500 mM Tris, 10-20 mM EDTA, 5% SDS, 200-500 mM NaCl, and 5-20 U/ml Proteinase K.

**Further,** in S21', the washing is performed using 0.1 × SSC.

Further, in S22, an incubation temperature is 50-60 °C.

Further, the incubation temperature is 53-58 °C.

Further, in S22, an incubation time is 5-15 min.

Further, the incubation time is 8-12 min.

Further, in S22, after incubation is completed, the step further includes washing the incubated product with 0.1 × SSC 2-4 times, thereby obtaining the solid support with impurities removed.

Further, S3 comprises: using a targeting probe, performing target capture on the cDNA on the solid support with impurities removed to obtain a circular DNA molecule, and performing rolling circle amplification on the circular DNA molecule to obtain the amplification product, wherein the amplification product is the *in situ* sequencing library.

Further, the target capture comprises: applying at least one targeting probe to a chip with impurities removed to capture a target gene, wherein the targeting probe successively comprises, from 5' to 3': an upstream identification sequence, a backbone sequence, optionally a barcode sequence, and a downstream identification sequence, the upstream identification sequence and the downstream identification sequence are complementary with a cDNA sequence of the target gene, and the 5' end of the upstream identification sequence in the targeting probe and the 3' end of the downstream identification sequence in the targeting probe are brought into proximity and ligated by a DNA ligase, thereby forming a circular DNA molecule.

Further, the targeting probe is at least one selected from the following: cck-1 shown in SEQ ID NO:1, cck-2 shown in SEQ ID NO:2, cck-3 shown in SEQ ID NO:3, cck-4 shown in SEQ ID NO:4, and cck-5 shown in SEQ ID NO:5.

Further, in S11, a plurality of Poly T clusters are provided, a diameter of each Poly T cluster is 218-222 nm, and a center-to-center distance between adjacent clusters is 498-505 nm.

Further, S11 comprises: performing pre-cooling treatment by placing the solid support with surface-modified Poly T clusters into a microtome, thereby obtaining a pre-cooled solid support; attaching a frozen section sample to the pre-cooled solid support, and heating and drying the pre-cooled solid support to obtain the mounted sample.

Further, a temperature for the pre-cooling treatment is -25 °C to -18 °C, and a time for the pre-cooling treatment is 0.5-6 min.

Further, a temperature for the heating is ≤37 °C, and a time for the heating is 2-5 min.

Further, S12 comprises: placing the mounted sample in pre-cooled methanol to fix cell morphology in the sample; and performing the permeabilization treatment by adding a permeabilization solution dropwise to the sample fixed in methanol.

Further, a temperature of the pre-cooling methanol is ≤-20 °C.

Further, a fixation time is 20-40 min.

Further, the fixation time is 25-35 min.

Further, the permeabilization solution comprises 0.01%-1% pepsin and 0.01 M-1 M HCl.

Further, the permeabilization solution comprises 0.1%-0.5% pepsin and 0.01M-0.1M HCl.

Further, a temperature for the permeabilization treatment is 35-38 °C.

In order to implement the above objective, a second aspect of the present invention provides an *in situ* sequencing method. The method comprises: constructing a sequencing library with the above construction method; and performing *in situ* sequencing on the sequencing library.

In order to implement the above objective, a third aspect of the present invention provides a kit. The kit includes any one or more selected from the group of a solid support having an mRNA capture function, a permeabilization reagent for tissue or cell permeabilization, an mRNA reverse transcription reagent, a tissue removal solution, a targeting probe, and a ligase.

Further, the kit includes any one or more of the following: (a) the solid support having the mRNA capture function, wherein the solid support having the mRNA capture function is a solid support with surface-modified Poly T clusters, preferably, a plurality of Poly T clusters are provided, a diameter of each Poly T cluster is 218-222 nm, and a center-to-center distance between adjacent clusters is 498-505 nm; (b) the tissue removal solution, wherein the tissue removal solution includes Proteinase K, preferably, the tissue removal solution with a pH of 8.0 includes 200-500 mM Tris, 10-20 mM EDTA, 5%-10% Triton X-100, 200-500 mM NaCl, and 5-20 U/ml Proteinase K; alternatively, the tissue removal solution with a pH of 8.0 includes 200-500 mM Tris, 10-20 mM EDTA, 5% SDS, 200-500 mM NaCl, and 5-20 U/ml Proteinase K; (c) the permeabilization reagent, wherein the permeabilization reagent includes pepsin, and preferably, the permeabilization reagent includes 0.1%-0.5% pepsin and 0.01 M-0.1 M HCl; (d) the targeting probe, wherein the targeting probe is a padlock probe; and preferably, the sequence of the padlock probe is selected from at least one of the following: cck-1 shown in SEQ ID NO:1, cck-2 shown in SEQ ID NO:2, cck-3 shown in SEQ ID NO:3, cck-4 shown in SEQ ID NO:4, or cck-5 shown in SEQ ID NO:5; (e) the ligase, wherein the ligase is a DNA ligase; and (f) a PCR amplification reagent.

In order to implement the above objective, a fourth aspect of the present invention provides an application of the kit in *in situ* sequencing.

In the technical solutions of the present invention, a capture probe (e.g., Poly T) is designed on a solid support such as a silicon-based chip, a slide, etc., and since the capture probe can bind to the mRNA in the tissue or cell sample, the mRNA in the sample is immobilized on the solid support. Compared with the prior art, in the technical solutions of the present invention, a chemical cross-linking reagent is not required to be used to fix the mRNA in the sample, such that the problem of reduced reverse transcription efficiency caused by a cross-linking fixation operation is avoided.

Furthermore, in the preferred technical solutions of the present invention, after the mRNA in the sample is immobilized, the tissue or cell samples are removed actively, and then subsequent experimental operations are performed, such that the impact caused by the retention of samples in the efficiency of subsequent reactions is avoided, and adverse effects on final detection due to the residual sequencing-related dyes in the tissue are also avoided. Therefore, according to the present invention, the interference of background fluorescence can be reduced, and the reaction efficiency and accuracy of *in situ* sequencing are greatly improved. Furthermore, the solid support such as the silicon-based chip, the slide, etc., on which the tissue or cell sample is removed is used, such that a platform that is easier to operate is provided for subsequent biochemical reactions and imaging, facilitating the implementation of the sequencing method.

### Brief Description of the Drawings

The drawings, which form a part of the present application, are used to provide a further understanding of the present invention. The exemplary embodiments of the present invention and the description thereof are used to explain the present invention, but do not constitute improper limitations to the present invention. In the drawings:
Fig. 1 is a schematic principle diagram of an *in situ* sequencing method according to the present application.
Fig. 2 is a schematic diagram of microscopic observation of *in situ* sequencing of a mouse-derived cck gene obtained according to Embodiment 1 of the present application.

### Detailed Description of the Embodiments

It is to be noted that the embodiments in the present application and the features in the embodiments may be combined with one another without conflict. The present invention will be described below in detail with reference to the drawings and the embodiments.

As mentioned in the Background, conventional sample processing and library construction methods for *in situ* transcriptome sequencing exhibit the following disadvantages: (1) Generally, to fix RNA of a cell/tissue sample within cells, cross-linking is required before a library is constructed with the cell/tissue sample. Specifically, the cell/tissue sample is treated by formaldehyde or PFA, and a covalent bond is formed between proteins and nucleic acids in the sample, thereby "freezing" the interaction state of the proteins and the nucleic acids. However, cross-linking fixation will damage the sample and reduce the efficiency of reverse transcription, thereby affecting the detection sensitivity and accuracy. (2) During library construction, when performing reagent application, washing, etc., the retention of the tissue sample may affect the reaction efficiency and increase background fluorescence interference. Furthermore, sequencing-related dyes also tend to linger in the tissue to produce adverse effect on final results.

Therefore, the inventors of the present application made improvements on a sample processing and library construction method for *in situ* transcriptome sequencing. A chip modified with a Poly T probe cluster is used to perform capture on a sample, and chemical cross-linking agents, e.g., formaldehyde, are no longer used. Furthermore, after mRNA is reverse transcribed into cDNA, residual tissue or residual cells of the sample are removed. The above improvements may reduce damages of the chemical cross-linking agents to the sample during sample preparation, and improve subsequent reverse transcription efficiency and sequencing sensitivity. The removing of the sample tissue or cell may reduce the impact of the tissue or cell on reaction efficiency when treatment of reagent application or washing is performed on the sample, such that the interference of background fluorescence during detection is reduced, thereby improving the detection accuracy, and facilitating subsequent on-chip biochemical reactions and imaging. On the basis of the above research results, the applicant has proposed a series of protection solutions in the present application.

A first typical implementation of the present invention provides a method for constructing an *in situ* sequencing library. The construction method includes: S1, placing a sample on a solid support having an mRNA capture function, and then performing non-crosslinking fixation and capture on mRNA in the sample, wherein the sample is a tissue sample or a cell sample; S2, reverse transcribing the captured mRNA into cDNA, and removing residual tissue in the reverse-transcribed tissue sample or residual cells in the reverse-transcribed cell sample, thereby obtaining a solid support with impurities removed, wherein the cDNA is immobilized on the solid support with impurities removed; and S3, performing target capture and amplification on the cDNA on the solid support with impurities removed, thereby obtaining an amplification product, wherein the amplification product is an *in situ* sequencing library.

According to the method for constructing an *in situ* sequencing library of the present invention, a chip having an mRNA capture function is used to capture the sample, such that adverse effects on reverse transcription efficiency induced by damage caused by cross-linking fixation of the mRNA using a chemical cross-linking method are avoided. After the reverse transcription, the residual tissue in the tissue sample or residual cells in the cell sample are removed, such that adverse effects on the sensitivity of detection in the prior art can be eliminated when reagent application or washing is performed on the tissue or cell. For the *in situ* sequencing library obtained by the above-mentioned method, due to the improvement of reaction efficiency during reverse transcription and subsequent amplification reactions, detected results are more accurate.

In a preferred embodiment of the present application, the sample is a section sample. In a preferred embodiment, the section sample is a tissue section or a cell section.

In S1 of the construction method, the solid support having the mRNA capture function is preferably a solid support with surface-modified Poly T clusters.

The non-crosslinking fixation means that the sample is fixed on the solid support without using a cross-linking agent (which is a method used in the prior art). For example, the mRNA is immobilized on the solid support through complementary pairing (a manner of intermolecular forces) between poly T and ploy A.

The "cross-linking agent" as used in the present application refers to substance that promotes or regulates the formation of a covalent bond or ionic bond between polymer molecules. It is a small molecule compound, has two or more reactive terminals targeting specific groups (e.g., amino, sulfhydryl, etc.), and may be coupled to two or more molecules, respectively, such that these molecules are bound together (i.e., forming covalent bonds).

In the present invention, the sample is fixed on the solid support without using the cross-linking agent. For example, unlike the prior art which uses the following cross-linking agents to fix the sample before capturing the mRNA: a carbonyl compound, a N-hydroxysuccinimide ester compound, an imidoester compound, a maleimide compound, a halogenated acetyl compound, a dithiodipyridine compound, and/or carbodiimide compound. In the present invention, the sample is fixed by means of non-crosslinking fixation, and subsequent mRNA capture is performed using a solid support modified with Poly T clusters, thereby precluding damage to mRNA from chemical cross-linking.

In a preferred embodiment of the present application, in S1, the residual tissue in the reverse-transcribed tissue sample or the residual cells in the reverse-transcribed cell sample are removed using Proteinase K. The removing of the sample residues creates homogeneous reaction environment for subsequent reverse transcription and amplification, enhancing reaction efficiency.

The solid support may be any solid-phase carrier suitable for *in situ* sequencing. In a preferred embodiment of the present application, the solid support is a silicon-based planar support. Any silicon-based planar support that may provide an operation platform for the sample is suitable for the present invention, such as a silicon-based chip, a glass slide, etc.

In a preferred embodiment of the present application, S1 in the construction method includes: S11, attaching the sample to a solid support with surface-modified Poly T clusters, thereby obtaining a mounted sample; and S12, performing permeabilization treatment on the mounted sample to release the mRNA in the sample, and capturing the mRNA in the sample on the solid support through the Poly T clusters.

In a preferred embodiment of the present application, S2 in the construction method includes: S21, reverse transcribing the mRNA into the cDNA using the Poly T cluster as a primer; and S22, incubating the sample on the solid support with a tissue removal solution, to remove the residual tissue in the reverse-transcribed tissue sample or the residual cells in the reverse-transcribed cell sample, thereby obtaining the solid support with impurities removed, wherein the cDNA is immobilized on the solid support with impurities removed. The tissue or cells of the reverse-transcribed sample on the solid support are removed to eliminate the interference of the tissue or cells to the amplification reaction during subsequent sample amplification, such that subsequent reaction efficiency is improved, and the quality of the obtained sequencing library is improved.

In a preferred embodiment of the present application, in S22, the tissue removal solution with a pH of 8.0 comprises 200-500 mM Tris, 10-20 mM EDTA, 5%-10% Triton X-100, 200-500 mM NaCl, and 5-20 U/ml Proteinase K; alternatively, the tissue removal solution with a pH of 8.0 comprises 200-500 mM Tris, 10-20 mM EDTA, 5% SDS, 200-500 mM NaCl, and 5-20 U/ml Proteinase K. A temperature for incubating the sample on the solid support with the tissue removal solution is 50-60 °C, and an incubation time is 5-15 min. In a preferred embodiment of the present application, the incubation temperature is 53-58 °C, and the incubation time is 8-12 min. The purpose of removing the residual tissue in the tissue sample or the residual cells in the cell sample is achieved with the tissue removal solution, such that the tissue or cell may be degraded and removed without destroying the biochemical properties of cDNA. Therefore, in order to achieve optimal reaction removal effects and efficiency, the reaction temperature and time need to be selected appropriately.

In order to further improve the efficiency of the subsequent amplification reaction after target capture, in some preferred embodiments, between S21 and S22, the method further includes S21' of washing residual reverse transcription-related reagents from the reverse-transcribed tissue sample or reverse-transcribed cell sample. The washing operation is to wash off residual liquid of the reverse transcription reaction, such that any reagent that may eliminate residual reagents is suitable for the present application. In a preferred embodiment of the present application, a reagent used for the washing is 0.1 × SSC.The reagent is a sodium citrate buffer, which is a wash buffer commonly used in experiments.

In order to further improve the efficiency of the subsequent target capture and amplification reactions, in some preferred embodiments, after incubation in S22 is completed, the tissue removal solution and the detached tissue or cells on the incubated solid support need to be washed, such that any reagent that may eliminate the residue reagents and the tissue or cells is suitable for the present application. In a preferred embodiment of the present application, the incubated solid support is washed 2-4 times with 0.1 × SSC, thereby obtaining the solid support with impurities removed. The efficiency of a subsequent rolling circle amplification reaction using the cDNA on the solid support with impurities removed is higher.

In order to further amplify a single molecule of a targeting sequence during library construction, in some preferred embodiments, S3 in the construction method includes: performing target capture using a targeting probe on the cDNA on the solid support with impurities removed to obtaining a circular DNA molecule, and performing rolling circle amplification on the circular DNA molecule to obtain the amplification product, wherein the amplification product is the *in situ* sequencing library.

In some preferred embodiments, the target capture includes: applying at least one targeting probe to a chip with impurities removed to capture a target gene, wherein the targeting probe successively includes, from 5' to 3' : an upstream identification sequence, a backbone sequence, optionally a barcode sequence, and a downstream identification sequence, the upstream identification sequence and the downstream identification sequence are complementary with a cDNA sequence of the target gene, and the 5' end of the upstream identification sequence in the targeting probe and the 3' end of the downstream identification sequence in the targeting probe are brought into proximity and ligated by a DNA ligase, thereby forming a circular DNA molecule. The targeting probe is a key point of *in situ* sequencing technology, forms a circular template *in situ* and enables rolling circle amplification through complementary hybridization of the upstream identification sequence and the downstream identification sequence to a target sequence. Subsequently, the barcode sequence of the probe is identified with fluorescence dyes that may identify four types of bases, thereby indicating what gene is bound at the spatial position.

In S3, the targeting probe is at least one selected from the following: cck-1 shown in SEQ ID NO:1, cck-2 shown in SEQ ID NO:2, cck-3 shown in SEQ ID NO:3, cck-4 shown in SEQ ID NO:4, and cck-5 shown in SEQ ID NO:5. In the present application, it confirms that using the targeting probe to amplify a cholecystokinin gene (cck) in a hippocampal region of a mouse brain in the sample, construction and *in situ* sequencing of the *in situ* sequencing library of the present application can be realized.

In order to further improve the resolution of *in situ* sequencing, in some preferred embodiments, in S11, a plurality of Poly T clusters on the solid support modified with the Poly T clusters are provided, a diameter of each Poly T cluster is 218-222 nm, and a center-to-center distance between adjacent clusters is 498-505 nm. The purpose of the Poly T clusters carried on the solid support in the present application is to capture the mRNA in the sample without using a chemical cross-linking agent. This requires that the diameter and density of the Poly T clusters be optimized to achieve optimal binding affinity of the Poly A tails of the mRNA with the clusters. Insufficient density compromises capture efficiency, while excessive density impedes the attachment of the sample to the solid support and causes unnecessary waste. The diameter of a Poly T cluster determines the number of probes in the single cluster, the center-to-center distance is a distance between the centers of two adjacent clusters, and the distance determines the final imaging resolution and the total probe density. If the center-to-center distance is closer, the probe density is higher, the image resolution is higher, and imaging is clearer.

In order to further reduce sample damages, in some preferred embodiments, S11 includes: performing pre-cooling treatment by placing the solid support with surface-modified Poly T clusters into a microtome, thereby obtaining a pre-cooled solid support; and attaching a frozen section sample to the pre-cooled solid support, and heating and drying the pre-cooled solid support to obtain the mounted sample. Sample processing requires a low-temperature environment, therefore, to prevent temperature damages from the sample before attachment, the solid support needs to be pre-cooled to a temperature similar to that of the sample, and then the sample and the Poly T on the solid support achieves complementary binding through baking, thereby obtaining the mounted chip. In a preferred embodiment of the present application, a temperature for the pre-cooling treatment is -25 °C to -18 °C, and a time for the pre-cooling treatment is 0.5-6 min. Further, a temperature for baking (heating) is ≤37 °C, and a time for baking (heating) is 2-5 min.

In order to maintain the morphology of cells in the sample on the solid support relatively intact, in some preferred embodiments, S12 includes: placing the mounted sample in pre-cooled methanol to fix cell morphology in the sample; and performing the permeabilization treatment by adding a permeabilization solution dropwise to the fixed sample. The cells in the sample are maintained at good morphology with the powerful dehydration power of the methanol. Then the permeabilization treatment is performed on the cells to release the mRNA in the cells, facilitating subsequent use of the solid support carrying a capture probe to capture and fix the mRNA. In a preferred embodiment of the present application, a pre-cooling temperature of the methanol is ≤-20 °C. An fixation time is 20-40 min, and more preferably, the fixation time is 25-35 min. The permeabilization solution includes 0.01%-1% pepsin and 0.01 M-1 M HCl, and more preferably, the permeabilization solution includes 0.1%-0.5% pepsin and 0.01 M-0.1 M HCl. A temperature for the permeabilization treatment is 35-38 °C.

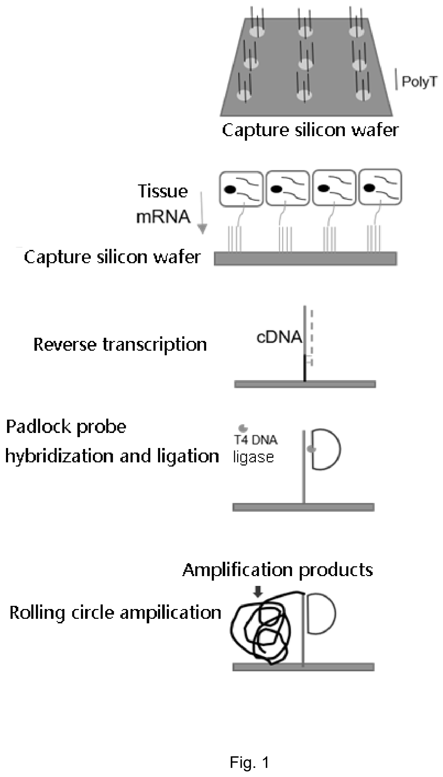

Fig. 1 is a schematic principle diagram of an *in situ* sequencing method according to the present invention. In a preferred embodiment, the size of a single poly T cluster on a capture silicon wafer is about 220 nm, and the distance between two clusters is about 500 nm. The tissue is sectioned (a thickness being about 10 µm) and attached to the capture silicon wafer, fixed in -20 °C methanol for 20 min, and then subjected to permeabilization treatment, such that the mRNA inside the tissue is released and captured by the probe on the capture silicon wafer. After being captured by the probe, the mRNA is subjected to a reverse transcription reaction to synthesize the cDNA; then a padlock probe is used for target capture, and a ligase (e.g., a T4 ligase) is used to fill in the gap; and finally, a polymerase (e.g., Phi29DNA) is used to perform an amplification reaction, thereby obtaining the amplification product, which is the *in situ* sequencing library.

A second typical implementation of the present invention provides an *in situ* sequencing method. The sequencing method includes: constructing a sequencing library with the construction method; and performing *in situ* sequencing on the sequencing library. The *in situ* sequencing method of the present invention causes less damage to mRNA of a sample. Furthermore, since without cross-linked fixation through a cross-linking agent, the efficiency of a reverse transcription reaction of the mRNA of the sample is higher. Furthermore, the *in situ* sequencing method of the present invention also has the advantages of being free of interference such as impurities, being good in imaging effect, and being high in resolution.

When a library is constructed, and a targeting probe carries a barcode sequence, in the *in situ* sequencing method of the present application, a complete barcode sequence of each amplification product may be obtained by decoding different barcode sequences in the *in situ* sequencing library, thereby obtaining the type of target RNAs detected by different amplification products, obtaining *in situ* expression information of different target RNAs, and realizing multiplex RNA *in situ* detection.

A third typical implementation of the present invention provides a kit. The kit includes any one or more selected from the group consisting of a solid support having an mRNA capture function, a permeabilization reagent for tissue or cell permeabilization, an mRNA reverse transcription reagent, a tissue removal solution, a targeting probe, and a ligase.

Any reagent suitable for the above kit components may be used in the kit. In a preferred embodiment, the kit includes any one or more of the following: (a) the solid support having the mRNA capture function, wherein the solid support having the mRNA capture function is a solid support with surface-modified Poly T clusters. In a preferred embodiment, a plurality of Poly T clusters are provided, a diameter of each Poly T cluster is 218-222 nm, and a center-to-center distance between adjacent clusters is 498-505 nm; (b) the tissue removal solution, wherein the tissue removal solution comprises Proteinase K, preferably, the tissue removal solution with a pH of 8.0 comprises 200-500 mM Tris, 10-20 mM EDTA, 5%-10% Triton X-100, 200-500 mM NaCl, and 5-20 U/ml Proteinase K; alternatively, the tissue removal solution with a pH of 8.0 comprises 200-500 mM Tris, 10-20 mM EDTA, 5% SDS, 200-500 mM NaCl, and 5-20 U/ml Proteinase K; (c) the permeabilization reagent, wherein the permeabilization reagent includes pepsin. In a preferred embodiment, the permeabilization reagent includes 0.1 %-0.5% pepsin and 0.01 M-0.1 M HCl; (d) the targeting probe, wherein the targeting probe is a padlock probe. In a preferred embodiment, the sequence of the padlock probe is selected from at least one of the following: cck-1 shown in SEQ ID NO: 1, cck-2 shown in SEQ ID NO: 2, cck-3 shown in SEQ ID NO: 3, cck-4 shown in SEQ ID NO: 4, and cck-5 shown in SEQ ID NO: 5; (e) the ligase, wherein the ligase is a DNA ligase; and (f) a PCR amplification reagent.

In a fourth typical implementation of the present invention provides an application of the kit in *in situ* sequencing. *In situ* sequencing is performed with the kit containing the above components, such that the reaction efficiency and accuracy of *in situ* sequencing can be greatly improved.

The present application is further described in detail below with reference to specific embodiments, and the embodiments cannot be construed as limiting the scope of protection claimed in the present application.

Embodiment 1 *in situ* sequencing was performed using a coronal section in a hippocampal region of a mouse brain as an example.
1. Sample and microtome treatment
   (1) Pre-cooling (-20 °C) of a cryostat chamber and pre-cooling (-10 °C-15 °C, adjusted according to an actual operation process) of a specimen head were performed.
   (2) A brush, a blade, tweezers, etc. were placed in the -20 °C chamber in advance for pre-cooling.
   (3) A tissue block was taken out from a -80 °C refrigerator and placed in the cryostat to equilibrate for 30 min.
   (4) The mouse brain tissue block (from Guangdong Medical Laboratory Animal Center) was trimmed, the sectioning area was less than 1 cm×1 cm, and excessive OCT embedding agent (Sakura, 4583) around the tissue block was cut off.
   (5) An OCT embedding agent was used to immobilize the tissue block on a specimen disc.
   (6) The tissue block was slightly treated as needed, and then sectioning was performed.
2. Chip treatment and tissue mounting
   (1) Methanol pre-cooling: methanol (with a use amount of 1 mL/well) was added in a 24-well plate, the 24-well plate was capped and placed in the microtome for pre-cooling, and a pre-cooling time did not exceed 30 min.
   (2) Chip taken: a chip was clipped from the 24-well plate using the tweezers and placed in a new 24-well plate, and a chip number was recorded; and attention was required to be paid to the front and back of the chip, the front of the chip should not be scratched.
   (3) A temperature of a slide baking machine was adjusted to 37 °C in advance.
   (4) For liquid-soaked chips: washing was performed twice with 400 µL of nuclease free water, and liquid on the surface of the chip was absorbed and discarded; and the back of the chip was placed on the slide baking machine and baked for 1 min at 37 °C, and when there was no residual liquid or corrugated texture on the surface of the chip, mounting might be prepared.
   (5) For dried chips: it was checked that there was no slicing debris or corrugated white texture on the surface of the chip (if there were slicing debris and corrugated white textures, washing was performed twice with nuclease free water and drying was performed), the surface of the chip was maintained dried or dried for 1 min at 37 °C, and then the mounting might be prepared.
   (6) Cold mounting: the chip was placed in the microtome with the front facing upward, and pre-cooled for 30 s-6 min (a pre-cooling time should not be too long, so as not to produce water mist on the surface of the chip; and the pre-cooling time should not be too short, so as not to prevent the chip from reaching a pre-cooling temperature); a frozen section was carefully covered over the center of the chip and tried to be successfully placed once, the chip was heated with a finger pulp on the backside to allow the section to adhere to the chip, and the chip was rapidly placed at 37 °C and baked for 3 min.
3. Tissue fixation and permeabilization
   (1) The chip dried in the previous step was placed in a methanol solution pre-cooled at -20 °C and fixed for 30 min.
   (2) 0.01 M HCl was prepared 5 min in advance, HCl was gradient-diluted to 0.01 M according to the concentration of the HCl, and at least 2 mL of 0.01 M HCl was prepared for later use.
   (3) Permeabilization reagent preparation: each tube of PR Enzyme in a dry powder state was dissolved with 1 mL of the 0.01 M HCl, then a pipette was used for blowing and well mixing, thereby obtaining a solution with a storage concentration of 10×, and then the solution was diluted10 times with 0.01 M HCl to reach a working solution concentration (a use volume of the working solution was 100 µL/chip).
   (4) A temperature of an incubator was adjusted to 37 °C.
   (5) The fixed tissue chip was taken from the methanol, the methanol from the back of and around the chip was absorbed with dust-free paper, the chip was placed in a culture dish pasted with a sealing film, and after the methanol was volatilized and the surface of the chip was dried , the next reaction was then performed.
   (6) The culture dish in which the chip was placed was transferred in a 37 °C incubator, and the permeabilization reagent was added dropwise, with a use amount of 100 µL/ chip. A tissue permeabilization time range was 0-30 min, and 12 min was used in this experiment.
   (7) The permeabilization reagent on the surface of the chip was absorbed, and 0.1 × SSC (containing 5% RI) was added, with a use amount of 100 µL/chip.
   (8) The liquid on the surface of the chip was absorbed, the liquid from the back of and around the chip was absorbed with dust-free paper, and a reaction solution of the next step was immediately added.
4. Reverse transcription reaction

A reverse transcription reaction mixed solution (RT Mix, from BGI Stereo-seq transcriptome kit, Catalog Number: 101KT114) was prepared according to the following reaction system.

**Table 1:**

| Reagent | Use amount (µL) |
|---|---|
| Reverse Transcriptase Reagent | 80 |
| RT Additive | 5 |
| RT Oligo | 5 |
| RNase Inhibitor | 5 |
| Reverse Transcriptase | 5 |
| Total | 100 |

The chip was horizontally placed in a culture dish pasted with a sealing film, 100 µL of the RT Mix was gently added dropwise from a corner of the chip, taking care that the liquid should evenly cover the entire chip; and then the culture dish in which the chip was placed was sealed with the sealing film, and a reaction was performed for 1 h or longer in a 42 °C incubator.

### 5. Tissue removal

(1) The temperature of the incubator was adjusted to 55 °C.
**(2) The RT** Mix on the surface of the chip was absorbed, the chip was transferred to the 24-well plate, and 0.1 × SSC (from BGI Stereo-seq transcriptome kit, Catalog Number: 101KT114) was used to wash the chip once (with a use amount of 400 µL/well).
(3) 400 µL of a tissue removal solution (pH 8.0, 200-500 mM Tris, 10-20 mM EDTA, 5%-10% Triton X-100, 200-500 mM NaCl, and 5-20 U/ml Proteinase K) was added and then placed in the 55 °C incubator for reaction for 10-30 min.
(4) The tissue removal solution was absorbed and discarded, and 0.1 × SSC was used to wash the chip repeatedly for twice (with a use amount of 400 µL/well).
(5) The washed chip was transferred into a new reaction well for the next reaction.

### 6. Targeting probe hybridization

A hybridization reaction mixed solution was prepared according to the following reaction system.

**Table 2:**

| Reagent | Storage concentration | Final concentration in the system | Use amount (µL) |
|---|---|---|---|
| NF-H₂O | - | - | 59 |
| T4 ligase buffer (NEB, B0202A) | 10X | 1X | 10 |
| KCl | 1 M | 50 mM | 5 |
| Formamide (VWR, 0606) | 100% | 20% | 10 |
| Padlock probe (synthesized by BGI Write) | 100 µM | 100 nM (total) | 1 |
| BSA (Sigma-Aldrich, A1933) | 20 µg/µL | 0.2 µg/µL | 1 |
| T4 DNA ligase (NEB, M0202M) | 5 U/µL | 0.5 U/µL | 10 |
| RNase H (NEB, M0293L) | 5 U/µL | 0.4 U/µL | 4 |
| Total | - | - | 100 |

The chip was horizontally placed in a culture dish pasted with a sealing film, 100 µL of the above prepared hybridization reaction mixed solution was gently added dropwise from a corner of the chip, taking care that the liquid should evenly cover the entire chip; and then the culture dish in which the chip was placed was sealed with the sealing film, a reaction was performed for 30 min in a 37 °C incubator, and then the culture dish was transferred in a 45 °C incubator for reaction for 90 min.

Sequences of the padlock probes were as follows:

**Table 3:**

| Probe name | Sequence (5'-3') | Modification |
|---|---|---|
| cck-1 | 5'-CCTGCTTGGAGGAGGTGGAACTGATTCCTTTGACTCACATTG CGTCCTATCTTCTTTGGAGCCTGTCTATTTAGTTAGTGGGCCAGC GTCTTGGC-3' (SEQ ID NO: 1) | 5' terminal phosphorylation |
| cck-2 | 5'-GGATTTTGGCCTjGCGCAGTGCTGATTCCTTTGACTCACATTG CGTCCTATCTTCTTTGGAGCCTGTCTATTTAGTCGGGACTACATG GGCTGGAT-3' (SEQ ID NO: 2) | 5' terminal phosphorylation |
| cck-3 | 5'-AGCCTTCTCCGCTGGAACTCCTGATTCCTTTGACTCACATTGC GTCCTATCTTCTTTGGAGCCTGTCTATTTAGTCCTCAACTTAGCT GGACTGC-3' (SEQ ID NO: 3) | 5' terminal phosphorylation |
| cck-4 | 5'-GGCAGTCCTAGCTGCTGGCGCTGATTCCTTTGACTCACATTG CGTCCTATCTTCTTTGGAGCCTGTCTATTTAGTGTATGTCTGTGC GTGGTGAT-3' (SEQ ID NO: 4) | 5' terminal phosphorylation |
| cck-5 | 5’-TTGAGTATCTATTTATTAAGCTGATTCCTTTGACTCACATTGCGT CCTATCTTCTTTGGAGCCTGTCTATTTAGTCGCCTCTAATGTCTG ACGTT-3’ (SEQ ID NO: 5) | 5' terminal phosphorylation |

### 7. RCA

An RCA reaction mixed solution was prepared according to the following reaction system.

**Table 4:**

| Reagent | Storage concentration | Final concentration in the system | Use amount (µL) |
|---|---|---|---|
| N F-H₂O | - | - | 33 |
| Phi29 buffer (NEB, B0269) | 10X | 1X | 10 |
| Glycerol | 50% | 5% | 10 |
| dNTPs Mix | 25 mM | 0.25 mM | 1 |
| 5-[3-Aminoallyl]-2'-deoxyuridine-5'-triphosphate sodium salt (Jena Bioscience, NU-803XL) | 20 mM | 20 µM | 0.1 |
| BSA (NEB, B9000S) | 20 µg/µL | 0.2 µg/µL | 1 |
| Exonuclease I (NEB, M0293L) | 20 U/µL | 0.4 U/µL | 2 |
| Phi29 polymerase (NEB, M0269) | 10 U/µL | 1 U/µL | 5 |
| Total | | | 100 |

The chip was horizontally placed in a culture dish pasted with a sealing film, 100 µL of the above prepared RCA reaction mixed solution was gently added dropwise from a corner of the chip, taking care that the liquid should evenly cover the entire chip; and then the culture dish in which the chip was placed was sealed with the sealing film, a reaction was performed for 4 h in a 37 °C incubator, and then the culture dish was transferred in a 30 °C incubator for reaction overnight.

After the overnight reaction, treatment was performed for 1 hour with 1 mM PEGylated bis (sulfosuccinimidyl)suberate (BS(PEG)9, Thermo Scientific, 21582), thereby promoting the fixation of an RCA product on the chip, and then the reaction was terminated with Tris-HCl (pH8.0, 20 mM), thereby obtaining the RCA product fixed on the chip.

### 8. RCA product detection

The reaction solution on the chip was washed and discarded, and washing was performed twice with 2×SSC and 10% formamide. A detection mixture was prepared according to the following reaction system.

**Table 5:**

| Reagent | Storage concentration | Final concentration in the system | Use amount (µL) |
|---|---|---|---|
| N F-H₂O | - | - | 49.9 |
| 2 × hybridization buffer | × SSC, 40% formamide | 2 × SSC, 20% formamide | 50 |
| D probe (synthesized by BGI Write) | 100 µM | 100nM | 0.1 |
| Total | | | 100 |

The D probe sequence (SEQ ID NO: 6) was as follows: Cy5-GTCCTATCTTCTTTGGAGCC, with the 5' terminal being modified with a fluorescence dye Cy5.

**100** µL of the detection mixed solution was gently added dropwise from a corner of the chip, taking care that the liquid should evenly cover the entire chip; and then the culture dish in which the chip was placed was sealed with the sealing film, and the reaction was performed for 15 min-30 min at room temperature.

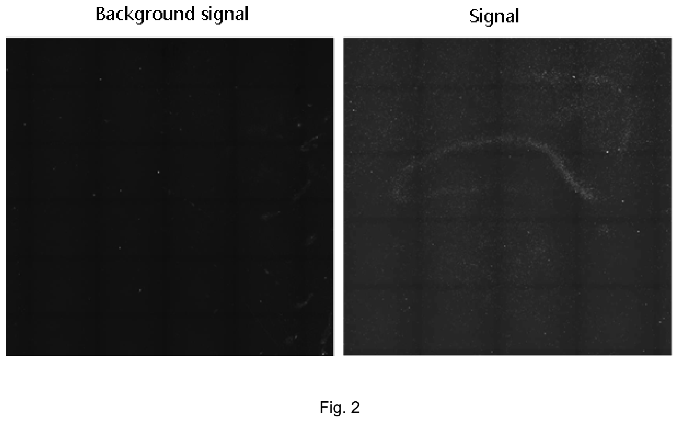

After the reaction was completed, washing was performed twice with 2×SSC and 10% formamide, then rinsing was performed twice with NF-H₂O, and after natural air-drying, imaging was performed under a wide-field microscope. An imaging result was shown in Fig. 2. The experimental result showed basically the same expression mode of the target gene (the cholecystokinin gene (cck) in the hippocampal region of the mouse brain) as *in situ* hybridization information from the Allen Brain database (gold standard). It indicated that, in the present invention, a library was successfully constructed, and *in situ* sequencing on the library was realized.

It may be seen from the above description that, in the above embodiments of the present invention, the following technical effects were realized: Applying the technical solutions of the present invention, the mRNA of the sample was captured using the solid support with Poly T without using chemical cross-linking reagents for treatment, and the tissue or cells of the sample were removed, it might be seen that the detected background fluorescence of the library constructed by the method was greatly reduced. Furthermore, a platform that is easier to conduct experiments is also provided for a microfluidic operation with the solid support such as a chip, etc., facilitating the implementation of the sequencing method.

The above are only the preferred embodiments of the present invention and are not intended to limit the present invention. For those skilled in the art, the present invention may have various modifications and variations. Any modifications, equivalent replacements, improvements and the like made within the spirit and principle of the present invention all fall within the scope of protection of the present invention.

## Claims

1. A method for constructing an *in situ* sequencing library, comprising:
S1, placing a sample on a solid support having an mRNA capture function, and then performing non-crosslinking fixation and capture on mRNA in the sample, wherein the sample is a tissue sample or a cell sample;
S2, reverse transcribing the captured mRNA into cDNA, and removing residual tissue in the reverse-transcribed tissue sample or residual cells in the reverse-transcribed cell sample, thereby obtaining a solid support with impurities removed, wherein the cDNA is immobilized on the solid support with impurities removed; and
S3, performing target capture and amplification on the cDNA on the solid support with impurities removed, thereby obtaining an amplification product, wherein the amplification product is an *in situ* sequencing library.

2. The method of claim 1, wherein the sample is a section sample.

3. The method of claim 2, wherein the section sample is a tissue section or a cell section.

4. The method of claim 1, wherein in S1, the solid support having the mRNA capture function is a solid support with surface-modified Poly T clusters.

5. The method of claim 1, wherein in S1, the non-crosslinking fixation means that the sample is fixed on the solid support without using a cross-linking agent.

6. The method of claim 5, wherein the cross-linking agent is a carbonyl compound, a N-hydroxysuccinimide ester compound, an imidoester compound, a maleimide compound, a halogenated acetyl compound, a dithiodipyridine compound, and/or a carbodiimide compound.

7. The method of claim 1, wherein in S2, the residual tissue in the reverse-transcribed tissue sample or the residual cells in the reverse-transcribed cell sample are removed with Proteinase K.

8. The method of claim 1, wherein the solid support is a silicon-based planar support.

9. The method of claim 1, wherein, S1 comprises:
S11, attaching the sample to a solid support with surface-modified Poly T clusters, thereby obtaining a mounted sample; and
S12, performing permeabilization treatment on the mounted sample to release the mRNA in the **sample,** and capturing the mRNA in the sample on the solid support through the Poly T clusters.

10. The method of claim 9, wherein S2 comprises:
S21, reverse transcribing the mRNA into the cDNA using the Poly T clusters as a primer; and
S22, incubating the sample on the solid support with a tissue removal solution, to remove the residual tissue in the reverse-transcribed tissue sample or the residual cells in the reverse-transcribed cell sample, thereby obtaining the solid support with impurities removed, wherein the cDNA is immobilized on the solid support with impurities removed.

11. The method of claim 10, wherein between S21 and S22, the method further comprises S21', washing residual reverse transcription-related reagents from the reverse-transcribed tissue sample or reverse-transcribed cell sample.

12. The method of claim 10, wherein the tissue removal solution with a pH of 8.0 comprises 200-500 mM Tris, 10-20 mM EDTA, 5%-10% Triton X-100, 200-500 mM NaCl, and 5-20 U/ml of Proteinase K;
or
the tissue removal solution with a pH of 8.0 comprises 200-500 mM Tris, 10-20 mM EDTA, 5% SDS, 200-500 mM NaCl, and 5-20 U/ml Proteinase K.

13. The method of claim 11, wherein in S21', the washing is performed using 0.1 × SSC.

14. The method of claim 10, wherein in S22, an incubation temperature is 50-60 °C.

15. The method of claim 14, wherein the incubation temperature is 53-58 °C.

16. The method of claim 10, wherein in S22, an incubation time is 5-15 min.

17. The method of claim 16, wherein the incubation time is 8-12 min.

18. The method of claim 10, wherein in S22, after incubation is completed, the step further comprises washing the incubated product with 0.1 × SSC 2-4 times, thereby obtaining the solid support with impurities removed.

19. The method of claim 1, wherein, S3 comprises:
using a targeting probe, performing target capture on the cDNA on the solid support with impurities removed to obtain a circular DNA molecule, and performing rolling circle amplification on the circular DNA molecule, thereby obtaining the amplification product, wherein the amplification product is the *in situ* sequencing library.

20. The method of claim 19, wherein the target capture comprises: applying at least one targeting probe to a chip with impurities removed to capture a target gene, wherein the targeting probe successively comprises, from 5' to 3': an upstream identification sequence, a backbone sequence, optionally a barcode sequence, and a downstream identification sequence; the upstream identification sequence and the downstream identification sequence are complementary with a cDNA sequence of the target gene, and the 5' end of the upstream identification sequence in the targeting probe and the 3' end of the downstream identification sequence in the targeting probe are brought into proximity and ligated by a DNA ligase, thereby forming a circular DNA molecule.

21. The method of claim 19, wherein the targeting probe is at least one selected from the following: cck-1 shown in SEQ ID NO:1, cck-2 shown in SEQ ID NO:2, cck-3 shown in SEQ ID NO:3, cck-4 shown in SEQ ID NO:4, and cck-5 shown in SEQ ID NO:5.

22. The method of claim 9, wherein in S11, a plurality of Poly T clusters are provided, a diameter of each Poly T cluster is 218-222 nm, and a center-to-center distance between adjacent clusters is 498-505 nm.

23. The method of claim 9, wherein, S11 comprises:
performing pre-cooling treatment by placing the solid support with surface-modified Poly T clusters into a microtome, thereby obtaining a pre-cooled solid support; and
attaching a frozen section sample to the pre-cooled solid support, and heating and drying the pre-cooled solid support, thereby obtaining the mounted sample.

24. The method of claim 23, wherein a temperature for the pre-cooling treatment is -25 °C to -18 °C, and a time for the pre-cooling treatment is 0.5-6 min.

25. The method of claim 23, wherein a temperature for the heating is ≤37 °C, and a time for the heating is 2-5 min.

26. The method of claim 9, wherein, S12 comprises:
placing the mounted sample in pre-cooled methanol to fix cell morphology in the sample; and
performing the permeabilization treatment by adding a permeabilization solution dropwise .

27. The method of claim 26, wherein a temperature of the pre-cooled methanol is ≤-20 °C.

28. The method of claim 26, wherein a fixation time is 20-40 min.

29. The method of claim 28, wherein the fixation time is 25-35 min.

30. The method of claim 26, wherein the permeabilization solution comprises 0.01%-1% pepsin and 0.01M-1M HCl.

31. The method of claim 30, wherein the permeabilization solution comprises 0.1%-0.5% pepsin and 0.01M-0.1M HCl.

32. The method of claim 26, wherein a temperature for the permeabilization treatment is 35-38 °C.

33. An *in situ* sequencing method, comprising:
using the method according to any one of claims 1 to 32 to construct a sequencing library; and
performing *in situ* sequencing on the sequencing library.

34. A kit, comprising any one or more selected from the group of: a solid support having an mRNA capture function, a permeabilization reagent for tissue or cell permeabilization, an mRNA reverse transcription reagent, a tissue removal solution, a targeting probe, and a ligase.

35. The kit of claim 34, comprising any one or more of the following:
(a) the solid support having the mRNA capture function, wherein the solid support having the mRNA capture function is a solid support with surface-modified Poly T clusters, preferably, a plurality of Poly T clusters are provided, a diameter of each Poly T cluster is 218-222 nm, and a center-to-center distance between adjacent clusters is 498-505 nm;
(b) the tissue removal solution, wherein the tissue removal solution comprises Proteinase K, preferably, the tissue removal solution with a pH of 8.0 comprises 200-500 mM Tris, 10-20 mM EDTA, 5%-10% Triton X-100, 200-500 mM NaCl, and 5-20 U/ml Proteinase K; alternatively, the tissue removal solution with a pH of 8.0 comprises 200-500 mM Tris, 10-20 mM EDTA, 5% SDS, 200-500 mM NaCl, and 5-20 U/ml Proteinase K;
(c) the permeabilization reagent, wherein the permeabilization reagent comprises pepsin, and preferably, the permeabilization reagent comprises 0.1%-0.5% pepsin and 0.01 M-0.1 M HCl;
(d) the targeting probe, wherein the targeting probe is a padlock probe; and preferably, the targeting probe is at least one selected from the following: cck-1 shown in SEQ ID NO:1, cck-2 shown in SEQ ID NO:2, cck-3 shown in SEQ ID NO:3, cck-4 shown in SEQ ID NO:4, and cck-5 shown in SEQ ID NO:5;
(e) the ligase, wherein the ligase is a DNA ligase; and
(f) a PCR amplification reagent.

36. An application for the kit of claim 34 or 35 in *in situ* sequencing.
